# EUROPEAN PATENT SPECIFICATION

(11) **EP 4 181 147 B1**
(45) Date of publication and mention of the grant of the patent: **17.12.2025**
(21) Application number: 20944346.4
(22) Date of filing: 08.07.2020
(51) Int. Cl.: G16B 30/10

(54) **INFORMATION PROCESSING PROGRAM, INFORMATION PROCESSING METHOD, AND INFORMATION PROCESSING DEVICE**
INFORMATIONSVERARBEITUNGSPROGRAMM, INFORMATIONSVERARBEITUNGSVERFAHREN UND INFORMATIONSVERARBEITUNGSVORRICHTUNG
PROGRAMME, PROCÉDÉ ET DISPOSITIF DE TRAITEMENT D'INFORMATIONS

(43) Date of publication of application: 17.05.2023
(73) Proprietor: FUJITSU LIMITED, Kawasaki-shi, Kanagawa 211-8588 (JP)
(72) Inventor: KATAOKA, Masahiro, Kawasaki-shi, Kanagawa 211-8588 (JP); MATSUMURA, Ryo, Kawasaki-shi, Kanagawa 211-8588 (JP); MOGUSHI, Kaoru, Minato-ku, Tokyo 105-7123 (JP)
(74) Representative: Hoffmann Eitle
(86) International application number: PCT/JP2020/026730
(87) International publication number: WO 2022/009342

(56) References cited:
- WO-A1-2014/080447
- WO-A1-2020/049748
- WO-A1-2020/049748
- US-A1- 2019 119 759
- US-A1- 2019 221 284

## Description

### FIELD

The present invention relates to an information processing program, an information processing method, and an information processing apparatus.

### BACKGROUND

In recent years, an impact of new viruses has been predicted to develop vaccines and the like by analyzing genomes that make up deoxyribonucleic acid (DNA) and ribonucleic acid (RNA) of humans and other organisms. Furthermore, research has been conducted for detecting, on the basis of the genomes, mutation (point mutation) such as cancer and gene abnormalities such as gene mutation, and for prophylaxes and diagnoses of diseases.

Specifically, there has been known a technique of storing base sequences of the human genome in association with positions and providing differences between individuals as useful semantic information. For example, positional information of the base sequence is obtained in response to request information of a genome analysis service or the like, and base sequence information to be associated with the obtained positional information is responded. There have been known techniques to reduce the time required for determining a frameshift of a mutation or detecting a potential gene mutation after a mutation point (WO 2020/049748 A1), to improve processing speed of retrieval and analysis of genome using compression and aggregation of variant information (US 2019/221284 A1) and to identify a number of mutational signature in patients with cancer (US 2019/119759 A1). These documents do not disclose the use of encoded codons for generating mutational signatures of different cancer types in the form of a statistical inverted index.

### SUMMARY

### TECHNICAL PROBLEM

However, a base sequence output from a sequencer is segmented for each several hundred bytes (B). Moreover, a data size of the base sequence of the human genome is 3 giga bytes (GB), which is significantly large.

Conventionally, since the base sequence of the personal genome is obtained in a segmented state, the segmented base sequences are connected. While the Burrows-Wheeler (BW) transform, block sorting, or the like is often used as a technique for the connecting, segmented parts are searched for and connected so that an analysis time is significantly long. Therefore, the length of the base sequence analysis time and the data size after the connection are the issues.

In one aspect, an object is to provide an information processing program, an information processing method, and an information processing apparatus capable of shortening a personal genome analysis time and reducing a data size.

### SOLUTION TO PROBLEM

The invention is set out in the appended set of claims.

### ADVANTAGEOUS EFFECTS OF INVENTION

According to one embodiment, it becomes possible to shorten a personal genome analysis time and to reduce a data size.

### BRIEF DESCRIPTION OF DRAWINGS

FIG. 1 is a diagram for explaining operation of an information processing apparatus according to a first embodiment.
FIG. 2 is a functional block diagram illustrating a functional configuration of the information processing apparatus according to the first embodiment.
FIG. 3 is a diagram illustrating an exemplary codon conversion table.
FIG. 4 is a diagram illustrating exemplary reference codon data.
FIG. 5 is a diagram illustrating an exemplary reference inverted index.
FIG. 6 is a diagram for explaining encoding of segmented genome data.
FIG. 7 is a diagram for explaining extraction of partial reference codon data.
FIG. 8 is a diagram for explaining codon sequences and narrowing down of the codon sequences using the reference inverted index.
FIG. 9 is a diagram for explaining narrowing down of codon sequences using the reference inverted index.
FIG. 10 is a diagram for explaining a reference genome, a personal genome, and an SNPs inverted index.
FIG. 11 is a diagram for explaining simultaneous execution of codon sequence comparison and SNPs inverted index generation.
FIG. 12 is a flowchart illustrating a process flow according to a first embodiment.
FIG. 13 is a diagram for explaining an exemplary system configuration according to a second embodiment.
FIG. 14 is a diagram for explaining a first causal relationship analysis at each hospital according to the second embodiment.
FIG. 15 is a diagram for explaining a second causal relationship analysis at each hospital according to the second embodiment.
FIG. 16 is a diagram for explaining an exemplary system configuration according to a third embodiment.
FIG. 17 is a diagram for explaining a first integrated analysis of causal relationships in an integrated analysis center according to the third embodiment.
FIG. 18 is a diagram for explaining a second integrated analysis of the causal relationships in the integrated analysis center according to the third embodiment.
FIG. 19 is a diagram for explaining an exemplary system configuration according to a fourth embodiment.
FIG. 20 is a diagram for explaining canceration diagnosis at each hospital using an integrated analysis result according to the fourth embodiment.
FIG. 21 is a diagram for explaining an exemplary hardware configuration.

### DESCRIPTION OF EMBODIMENTS

Hereinafter, embodiments of an information processing program, an information processing method, and an information processing apparatus according to the present invention will be described in detail with reference to the drawings. Note that the present invention is not limited by those embodiments. Furthermore, the individual embodiments may be appropriately combined with each other unless otherwise contradicted.

### [First Embodiment]

### [Description of Information Processing Apparatus 10]

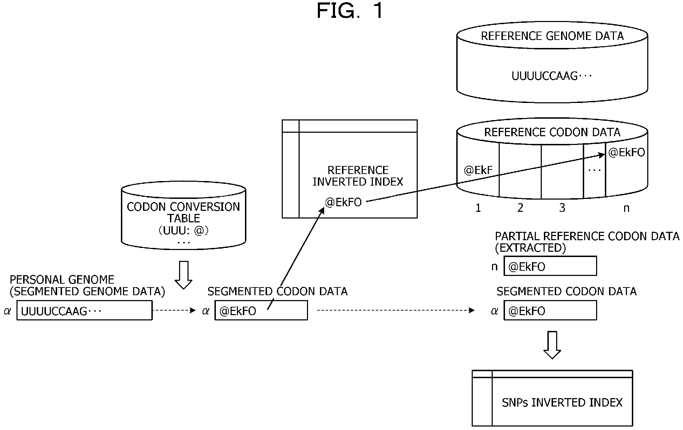

FIG. 1 is a diagram for explaining operation of an information processing apparatus 10 according to a first embodiment. The information processing apparatus 10 illustrated in FIG. 1 is an exemplary computer device that analyzes characteristics of a personal genome and achieves prophylaxes and diagnoses of diseases by analyzing base sequence data of the genome of the individual to be analyzed and identifying a sequence part different from the normal base sequence data as a reference. Note that, in the present embodiment, the base sequence data of the genome of the individual may be referred to as a "personal genome" or "personal genome data", and the normal base sequence data as a reference may be referred to as a "reference genome" or "reference genome data".

First, a genome is genetic information, which is a base sequence of DNA or RNA. Next, codons, which are three bases, determine amino acids, and multiple amino acids make up protein. Moreover, multiple proteins bind to form a primary structure, a secondary structure, and a tertiary (higher-order) structure.

Meanwhile, there are four types of DNA or RNA bases, which are denoted by symbols of "A", "G", "C", and "T" or "U". Furthermore, a group of three base sequences is called a "codon", and there are 64 kinds of them, which determine 20 kids of amino acids. Each of the amino acids is denoted by symbols of "A" to "Y". Multiple types of codons are associated with one amino acid. Accordingly, for example, an amino acid "alanine (Ala)" is associated with codons "GCU", "GCC", "GCA", and "GCG". It has the characteristic of being the same amino acid even if the third base is different.

As illustrated in FIG. 1, the information processing apparatus 10 retains a codon conversion table in which codons and compression codes (may be simply referred to as "codes" hereinafter) assigned to the codons are associated with each other. For example, in the codon conversion table, "UUU, @" or the like is associated as "codon, code".

Then, the information processing apparatus 10 generates reference codon data "@Ek..." obtained by encoding the reference genome data "UUU..." in codon units using the codon conversion table. Furthermore, the information processing apparatus 10 generates a bitmap-type reference inverted index in which the codon code and the appearance position in the reference codon data are associated with each other.

In such a state, the information processing apparatus 10 obtains segmented genome data a to η from a sequencer that performs sequencing of the personal genome. Then, the information processing apparatus 10 refers to the codon conversion table to encode, in codon units, each of the segmented genome data a to η in the state of being segmented, thereby generating segmented codon data a to η.

Then, the information processing apparatus 10 sequentially extracts partial reference codon data from the reference codon data using the reference inverted index for each of the segmented codon data a to η. By sequentially comparing the segmented codon data with the partial reference codon data in codon units, a single-nucleotide polymorphism (hereafter referred to as gene mutation) indicating a subtle difference in genetic information between individuals is detected, and a bitmap-type SNPs inverted index (gene mutation inverted index) in which a type and position of mutation are associated with each other is generated.

At this time, the information processing apparatus 10 narrows down the codon sequences corresponding to the segmented codon data using the reference inverted index without connecting the segmented codon data a to η, and extracts the partial reference codon data, whereby the generation of the SNPs inverted index may be speeded up. For example, the information processing apparatus 10 narrows down the position where encoded data "@, E, k, F, O" of the reference codon sequence "UUU, UCC, AAG, UCA, UGG" to be searched for, which is specified in advance, appears from the reference inverted index of the reference genome by searching for the longest-match string.

Here, the information processing apparatus 10 compares the segmented codon data with the extracted partial reference codon data in codon units, and detects gene mutation of different codons. Then, the information processing apparatus 10 initializes the inverted index to "0", and sets "1" only to bits corresponding to bases of the different codons and their positions, whereby an SNPs inverted index 20 may be generated without connecting all the segmented codon data.

In this manner, even in a case where the personal genome is segmented, the information processing apparatus 10 is enabled to analyze the gene mutation while it remains segmented, whereby the analysis time of the personal genome may be shortened.

### [Functional Configuration]

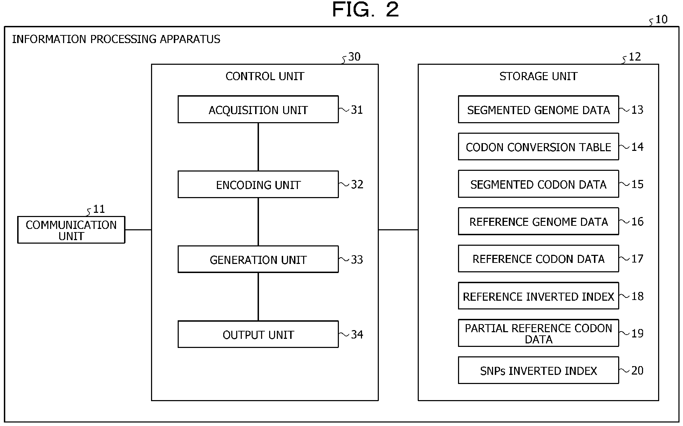

FIG. 2 is a functional block diagram illustrating a functional configuration of the information processing apparatus 10 according to the first embodiment. As illustrated in FIG. 2, the information processing apparatus 10 includes a communication unit 11, a storage unit 12, and a control unit 30.

The communication unit 11 is a processing unit that controls communication with another device, and is implemented by, for example, a communication interface or the like. For example, the communication unit 11 transmits/receives data to/from the sequencer, which is a providing source of the personal genome, and receives segmented genome data 13a to 13η segmented for each several hundred B.

The storage unit 12 is a processing unit that stores various types of data, various programs to be executed by the control unit 30, and the like, and is implemented by, for example, a memory, a hard disk, or the like. This storage unit 12 stores segmented genome data 13, a codon conversion table 14, segmented codon data 15, reference genome data 16, reference codon data 17, a reference inverted index 18, partial reference codon data 19, and the SNPs inverted index 20.

The segmented genome data 13 is segmented base sequence data obtained by segmenting the personal genome to be analyzed into a predetermined size. For example, the segmented genome data 13 is data including the segmented genome data 13a "UUU..." to the segmented genome data 13η"... C" generated from the personal genome "UUUUUCA...". This segmented genome data 13 is obtained by the control unit 30.

The codon conversion table 14 is information to be used at a time of encoding a base sequence, and stores codons and codes in association with each other. Specifically, the codon conversion table 14 is conversion information in which high-frequency codons with high appearance frequencies and codes assigned to the high-frequency codons are associated with each other.

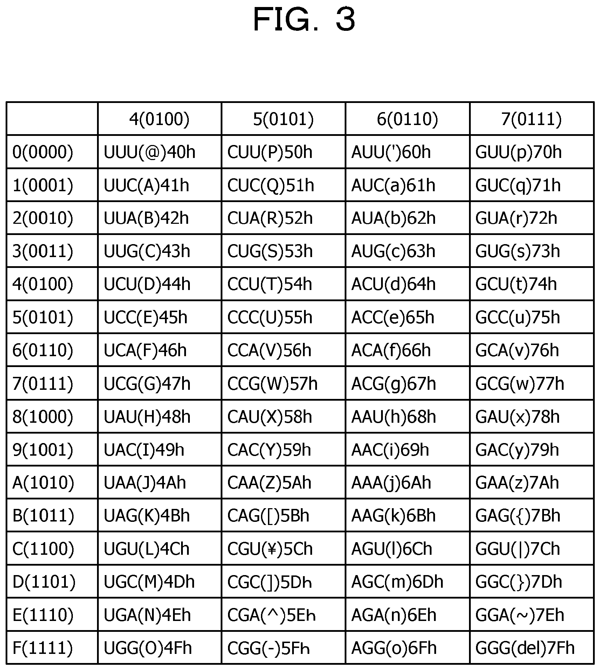

FIG. 3 is a diagram illustrating an example of the codon conversion table 14. As illustrated in FIG. 3, for example, a code of the codon "UUU" is "40h(01000000)". The reference "h" indicates a hexadecimal number. Note that, in the present embodiment, "40h(01000000)" described when the codon "UUU" is encoded is written as "UUU(40h)" or the like for convenience of explanation. Furthermore, "UUU(40h)" may be symbolized and written as "UUU(@)" or the like.

The reference genome data 16 is base sequence data of the human genome to be a reference. For example, the Japanese reference genome is made public by Tohoku University Tohoku Medical Megabank Organization. Note that the reference genome data 16 may be stored in advance, or may be obtained from a server or the like designated by the control unit 30.

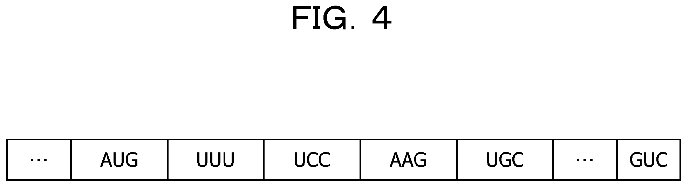

The reference codon data 17 is encoded data obtained by encoding the reference genome data 16 in codon units. FIG. 4 is a diagram illustrating an example of the reference codon data 17. As illustrated in FIG. 4, multiple codons are arranged in the reference codon data 17. Note that the reference codon data 17 may be stored in advance, or may be generated by the control unit 30.

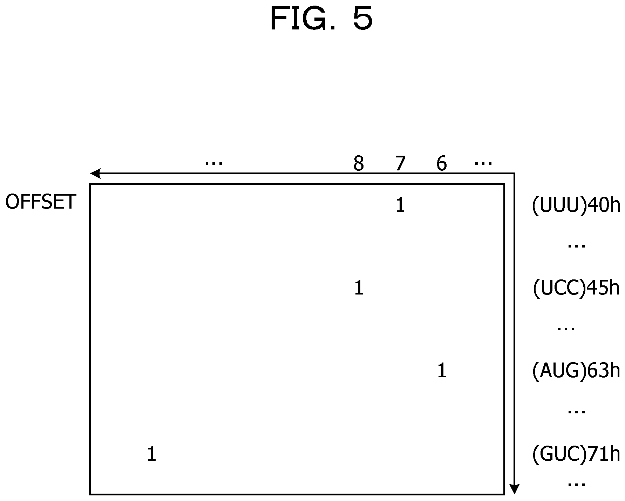

The reference inverted index 18 is a bitmap-type inverted index in which the codon code and the appearance position in the reference codon data 17 are associated with each other. FIG. 5 is a diagram illustrating an example of the reference inverted index 18.

As illustrated in FIG. 5, the horizontal axis of the reference inverted index 18 is an axis corresponding to an offset. The vertical axis of the reference inverted index 18 is an axis corresponding to a codon type (codon code). The reference inverted index 18 is indicated by a bitmap of "0" or "1", and all bitmaps are set to "0" in the initial state. For example, the offset of the top codon code of the reference inverted index 18 is set to "0". In a case where a codon code "(AUG)63h" is included at the seventh position from the top of the reference inverted index 18, a bit at a position where a column of an offset "6" of the reference inverted index 18 intersects with a row of the codon code "(AUG)63h" is set to "1". Note that the reference inverted index 18 may be stored in advance, or may be generated by the control unit 30.

The SNPs inverted index 20 is a bitmap-type inverted index of gene mutation for the personal genome. Specifically, the SNPs inverted index 20 is a bitmap-type inverted index in which each of the segmented codon data 15 is compared with the partial reference codon data 19 extracted from the reference codon data 17 and a type and position of different gene mutation are associated with each other. Note that the structure of the SNPs inverted index 20 is similar to that of the reference inverted index 18, and descriptions thereof will be omitted. For example, the SNPs inverted index 20 is provided with a bitmap for each type of predetermined SNPs such as the third base SNPs.

The control unit 30 is a processing unit that takes overall control of the information processing apparatus 10, and is, for example, a processor or the like. The control unit 30 includes an acquisition unit 31, an encoding unit 32, a generation unit 33, and an output unit 34. Note that the acquisition unit 31, the encoding unit 32, the generation unit 33, and the output unit 34 are implemented by an electronic circuit included in a processor, a process executed by the processor, or the like.

The acquisition unit 31 is a processing unit that obtains the segmented genome data 13. For example, the acquisition unit 31 obtains the segmented genome data 13 from a specified providing source, and stores it in the storage unit 12. Note that the acquisition unit 31 may receive the segmented genome data 13 transmitted from the providing source, or may obtain it periodically.

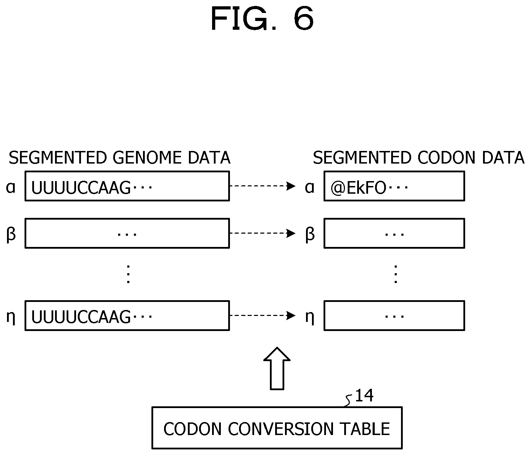

The encoding unit 32 is a processing unit that encodes the segmented genome data 13. FIG. 6 is a diagram for explaining encoding of the segmented genome data 13. As illustrated in FIG. 6, the encoding unit 32 encodes each of the segmented genome data 13a "UUU..." to the segmented genome data 13η "... C" included in the segmented genome data 13 to codons with three base symbols on the basis of the codon conversion table 14, thereby generating segmented codon data a ""UUU..." to segmented codon data η "... C".

At this time, the encoding unit 32 assigns a codon code to a three-base sequence registered in the codon conversion table 14, and encodes it.

The generation unit 33 is a processing unit that generates the SNPs inverted index 20. Specifically, in a case where the segmented genome data 13 of the personal genome of a certain individual is obtained, the generation unit 33 analyzes the segmented genome, and generates a bitmap-type SNPs inverted index 20 indicating gene mutation.

For example, the generation unit 33 sequentially extracts the partial reference codon data 19 from the reference codon data 17 using the reference inverted index 18 for each of the segmented codon data a to η, and sequentially compares it. Then, the generation unit 33 detects gene mutation included in each of the segmented codon data, sets "1" to a bit that associates a type and position of the gene mutation, generates the SNPs inverted index 20, and stores it in the storage unit 12.

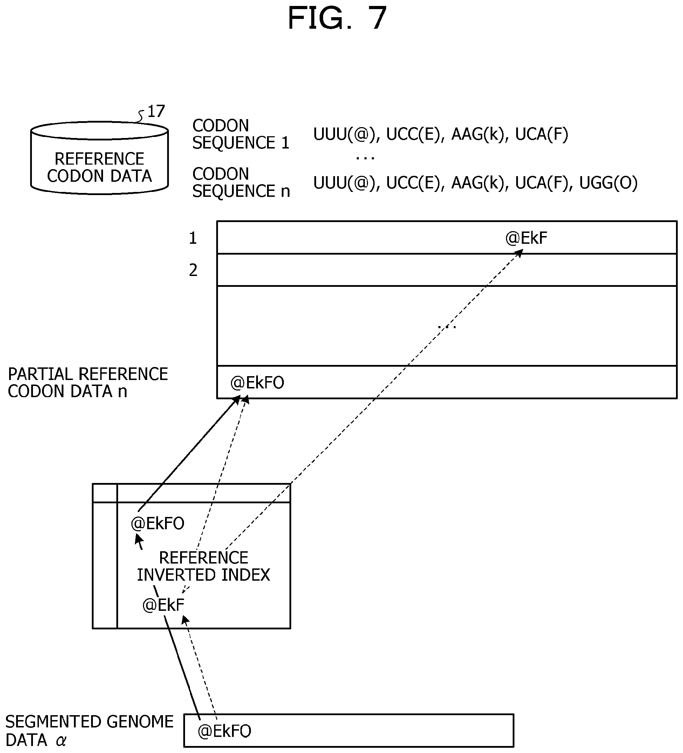

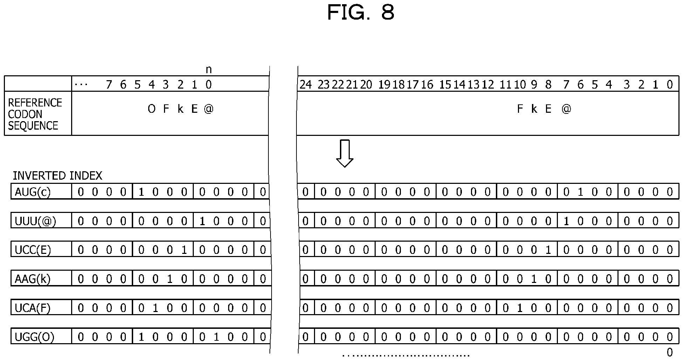

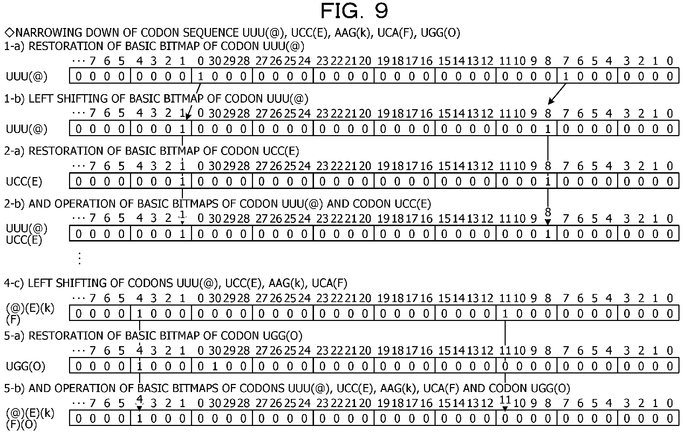

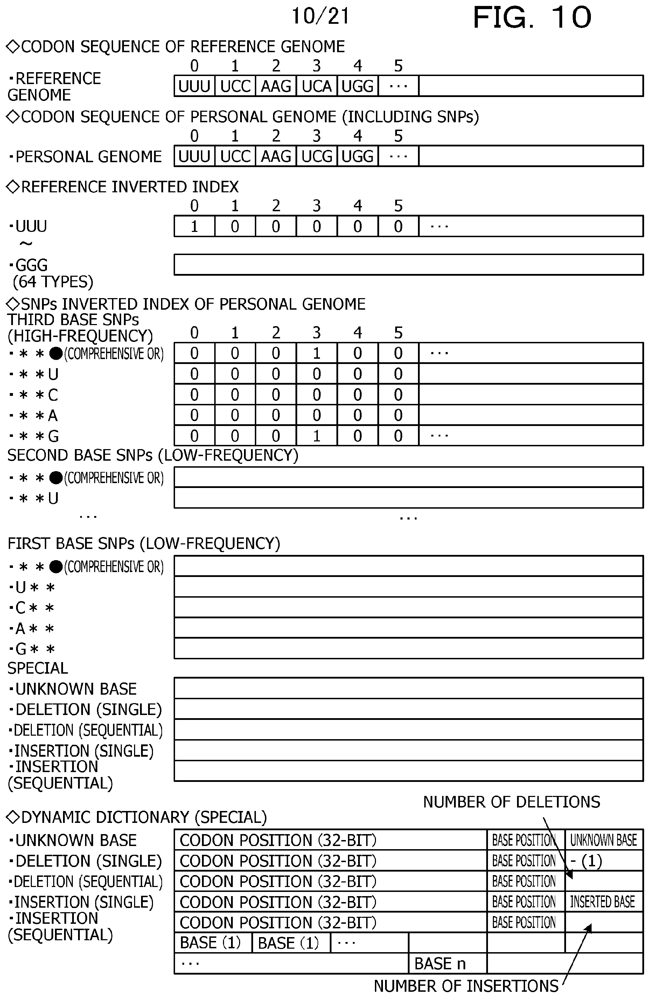

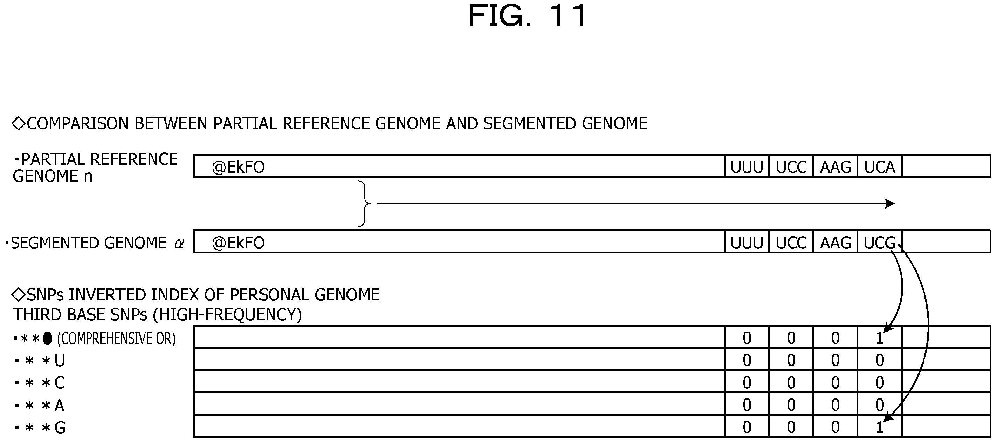

Here, the generation unit 33 may speed up the generation of the SNPs inverted index 20 by extracting the partial reference codon data 19 from the segmented codon data a to η using the reference inverted index 18. In view of the above, the extraction process and the generation of the SNPs inverted index 20 will be specifically described with reference to FIGs. 7 to 11. FIG. 7 is a diagram for explaining an outline of the extraction of the partial reference codon data, and FIG. 8 is a diagram for explaining codon sequences and narrowing down of the codon sequences using the reference inverted index 18. FIG. 9 is a diagram for explaining narrowing down of the codon sequences using the reference inverted index 18. FIG. 10 is a diagram for explaining the reference genome, the personal genome, and the SNPs inverted index 20. FIG. 11 is a diagram for explaining simultaneous execution of comparison of the codon sequences and generation of the SNPs inverted index 20.

As illustrated in FIG. 7, the generation unit 33 obtains the segmented codon data a to η. Subsequently, the generation unit 33 performs a longest-match string search on the reference codon data 17 with the codon sequence of the segmented codon data 15 as an input using the reference inverted index 18 generated in advance. As a result, the reference codon sequence (4) "UUU(@), UCC(E), AAG(k), UCA(F)" and the reference codon sequence (5) "UUU(@), UCC(E), AAG(k), UCA(F), UGG(O)", which are the reference codon sequence to be searched for (characteristic sequence of predetermined protein), are sequentially narrowed down. Then, the generation unit 33 may identify the partial reference codon data 19 corresponding to the segmented codon data 15 to extract it at high speed.

FIG. 8 illustrates an example of the reference inverted index 18 generated for the reference codon data 17. For example, since the codon code "UUU@" appears at the seventh offset, "1" is set to the seventh bit of the bitmap of the codon code "UUU@" in the reference codon data 17. Similarly, since the codon code "UGG(O)" appears at the 10th and 30th positions, "1" is set to each of the 10th and 30th bits of the bitmap of the codon code "UGG(O)" in the reference codon data 17.

An example of performing narrowing down using the reference inverted index 18 in this manner will be described with reference to FIG. 9. Specifically, the generation unit 33 performs bitmap shifting and AND operation for the codon sequence (4) "UUU(@), UCC(E), AAG(k), UCA(F)" and the codon sequence (5) "UUU(@), UCC(E), AAG(k), UCA(F), UGG(O)" with the reference inverted index 18. That is, the generation unit 33 identifies and extracts the codon sequence in which multiple "1"s are narrowed down to a single "1" in the logical operation of the bitmap of the reference inverted index 18.

Here, as an example, how the reference codon data 17 is narrowed down according to the codon sequence (4) "UUU(@), UCC(E), AAG(k), UCA(F)" using the reference inverted index 18 will be described with reference to FIG. 9. As illustrated in FIG. 9, the generation unit 33 refers to the reference inverted index 18 to obtain bitmaps corresponding to the individual codons "UUU(@)", "UCC(E)", "AAG(k)", and "UCA(F)". A bitmap of the codon code "UUU(@)" is referred to as a bitmap b_UUU. A bitmap of the codon code "UCC(E)" is referred to as a bitmap b_UCC. A bitmap of the codon code "AAG(k)" is referred to as a bitmap b_AAG. A bitmap of the codon code "UCA(F)" is referred to as a bitmap b_UCA.

The generation unit 33 obtains the bitmap b_UUU (see 1-a in FIG. 9), and shifts the bitmap b_UUU to the left, thereby generating a bitmap b20 (see 1-b in FIG. 9). The generation unit 33 obtains the bitmap b_UCC, and performs an AND operation on the bitmap b_UCC and the bitmap b20, thereby generating a bitmap b21 (see 2-a in FIG. 9). Since "1" stands at the offsets "8" and "n + 1" of the bitmap b21, it is found that the offsets 7 to 8 and n to n + 1 include the codon "UUU(@), UCC(E)" (see 2-b in FIG. 9).

In this manner, the left shifting and the AND operation are used to search for positions where "1" appears in succession. Specifically, the generation unit 33 shifts the bitmap b21 to the left to generate a bitmap b22. The generation unit 33 obtains the bitmap b_AAG, and performs an AND operation on the bitmap b_AAG and the bitmap b22, thereby generating a bitmap b23. Since "1" stands at the offsets "9" and "n + 2" of the bitmap b23, it is found that the offsets 7 to 9 and n to n + 2 include the codon "UUU(@), UCC(E), AAG(k)".

The generation unit 33 shifts the bitmap b23 to the left to generate a bitmap b24. The generation unit 33 obtains the bitmap b_UCA, and performs an AND operation on the bitmap b_UCA and the bitmap b24, thereby generating a bitmap b25. Since "1" stands at the offsets "10" and "n + 3" of the bitmap b25, it is found that the offsets 7 to 10 and n to n + 3 include the codon "UUU(@), UCC(E), AAG(k), UCA(F)".

Moreover, the generation unit 33 shifts the bitmap b25 to the left to generate a bitmap b26. A bitmap b_UGG corresponding to the codon UGG(O) is obtained for the codon sequence (5) "UUU(@), UCC(E), AAG(k), UCA(F), UGG(O)". An AND operation is performed on the bitmap b_UGG and the bitmap b26 to generate a bitmap b27. Since "1" stands only at the offset "n + 4" of the bitmap b27, it is found that the offsets n to n + 4 include the codon "UUU(@), UCC(E), AAG(k), UCA(F), UGG(O)" and multiple candidates have been narrowed down to one.

In this manner, the generation unit 33 executes the process illustrated in FIG. 9 to identify and extract the partial reference codon data 19 containing the codon sequence (5) "UUU(@), UCC(E), AAG(k), UCA(F), UGG(O)" in the reference codon data 17. The generation unit 33 repeatedly executes the process described above for other segmented codon data 15 as well to identify and extract the partial reference codon data 19 included in the reference codon data 17.

Next, the generation unit 33 compares the segmented codon data 15 of the personal genome with the partial reference codon data 19 extracted in FIG. 7 to detect gene mutation, and identifies a type and position thereof. Here, descriptions will be given using an example in which the position of the gene mutation is specified by a bit position (0, etc.). As illustrated in FIG. 10, the codon code of the reference genome (reference codon data 17) corresponding to bit positions "0, 1, 2, 3" is "UUU, UCC, AAG, UGA", and the codon code of the personal genome (segmented codon data 15) is "UUU, UCC, AAG, UGG".

In this case, the generation unit 33 sets "1" to the 0 bit position in advance in the bitmap (bitmap b_UUU) of the codon code "UUU@" of the reference inverted index 18.

Next, the SNPs inverted index 20 of the personal genome corresponding to the reference inverted index 18 will be described. As for the gene mutation type, U, C, A, G, and comprehensive bitmaps are provided for each of the third, second, and first bases according to the three bases of the codon. (The comprehensive bitmap may be omitted.) In general, gene mutation commonly occurs in the third base, and rarely occurs in the second base and the first base. Note that a dynamic dictionary storing bitmaps and detailed information associated with special gene mutation is also provided.

As illustrated in FIG. 11, the generation unit 33 compares the extracted partial reference codon data 19 with the segmented codon data 15 in codon units to detect different codons "UCA" and "UCG", and identifies the bitmap of "**G" and the position of the gene mutation in the third base. As a result, the generation unit 33 sets "1" to the corresponding bit positions of "comprehensive" and "**G" bitmaps of the third base as the SNPs inverted index 20.

That is, as illustrated in FIG. 11, at the time of comparing the reference genome with the personal genome, the generation unit 33 narrows down the positions of the reference codon sequences, and make a comparison from the narrowed down positions. Then, the generation unit 33 may detect a codon sequence partially different from the reference genome in the personal genome, and may identify a type and position of gene mutation. Therefore, the generation unit 33 is enabled to simultaneously execute the process of codon sequence comparison and the process of generating the SNPs inverted index 20 by extracting the partial reference codon data 19 using the reference inverted index 18 without connecting the segmented personal genome.

Returning to FIG. 2, the output unit 34 is a processing unit that outputs the SNPs inverted index 20 generated by the generation unit 33. For example, the output unit 34 displays and outputs the SNPs inverted index 20 on a predetermined display, and transmits the SNPs inverted index 20 to a predetermined destination.

### [Process Flow]

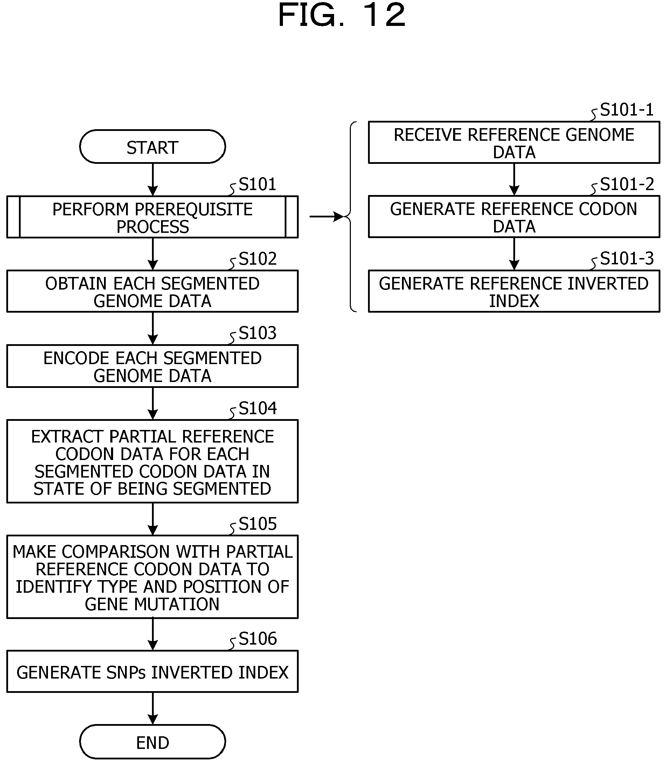

FIG. 12 is a flowchart illustrating a process flow according to the first embodiment. As illustrated in FIG. 12, the information processing apparatus 10 executes prerequisite process (S101). Specifically, the information processing apparatus 10 receives the reference genome data 16 (S101-1), and encodes (compresses) the reference genome data 16 in codon units on the basis of the codon conversion table 14 to generate the reference codon data 17 (S101-2). Then, the information processing apparatus 10 generates the reference inverted index 18 on the basis of the reference codon data 17 (S101-3).

Thereafter, the acquisition unit 31 obtains each of the segmented genome data (S102), and the encoding unit 32 encodes each of the segmented genome data in codon units on the basis of the codon conversion table 14 to generate each of the segmented codon data 15 (S103).

Then, the generation unit 33 extracts, using the reference inverted index 18, the partial reference codon data 19 corresponding to the individual segmented codon data 15 in the state of being segmented (S104). Thereafter, the generation unit 33 compares the extracted partial reference codon data 19 with each of the segmented codon data 15 to identify a type and position of gene mutation (S105), and generates the SNPs inverted index 20 (S106).

### [Effects]

As described above, the information processing apparatus 10 compresses and encodes the base sequence of the reference genome in codon units, and generates a bitmap-type inverted index corresponding to the codon. Furthermore, the information processing apparatus 10 compresses and encodes the segmented base sequences of the personal genome in codon units, searches for the longest-match string using the inverted index of the reference genome, narrows down the area, and extracts a partial reference genome corresponding to each of the segmented base sequences. At the same time, the information processing apparatus 10 compares the partial reference genome with the segmented personal genome in codon units to generate the bitmap-type SNPs inverted index. Therefore, the information processing apparatus 10 is enabled to analyze the gene mutation and generate SNPs inverted index by codon encoding without connecting the segmented personal genome, whereby it becomes possible to shorten the analysis time of the personal genome and to reduce the data size.

Note that, with regard to the reference inverted index associated with the 64 types of codons and their positions, the narrowing down may be speeded up by expanding the codons to N grams although the index size increases. For example, when expanded to 2 grams, the narrowing down is speeded up to 1/2 although the size increases from 64 types to 4,096 (64 x 64) types. Furthermore, in a similar manner to the text inverted index, the SNPs inverted index may also be hashed with adjacent prime numbers. Since each of the SNPs may be compressed to a capacity of 6 to 8 bits, the SNPs inverted index per person is approximately several kilo bytes (KB). Meanwhile, while the extraction of the partial reference codon data fails if the SNPs are included near the top of the segmented genome data, it is sufficient if the narrowing down is carried out again from the codon after the SNPs.

### [Second Embodiment]

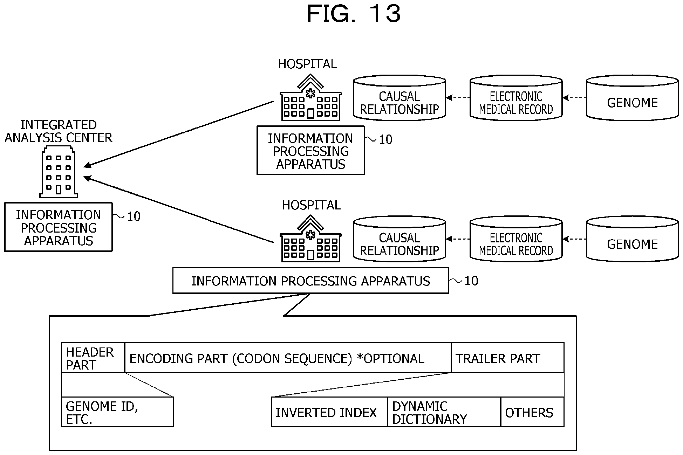

In a second embodiment, an example of being applied to canceration diagnosis at hospitals will be described. FIG. 13 is a diagram for explaining an exemplary system configuration according to the second embodiment. In a system illustrated in FIG. 13, an integrated analysis center is connected to individual hospitals in a mutually communicable manner via a network. Each of the integrated center and individual hospitals has an information processing apparatus 10 having the functions described in the first embodiment.

In such a system configuration, the information processing apparatus 10 of each of the hospitals analyzes the personal genome of a patient to generate an electronic medical record, and analyzes a causal relationship with cancer. Then, the information processing apparatus 10 of each of the hospitals transmits the causal relationship to the information processing apparatus 10 of the integrated analysis center. With this arrangement, the information processing apparatus 10 of the integrated analysis center is enabled to collect the causal relationships executed in the individual hospitals.

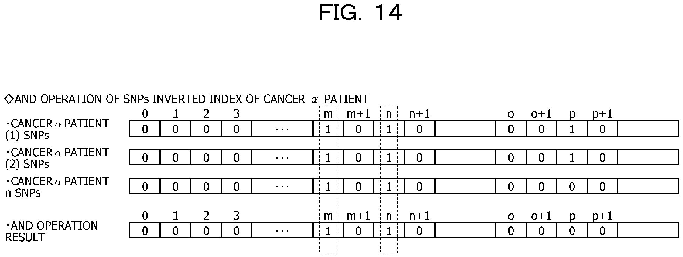

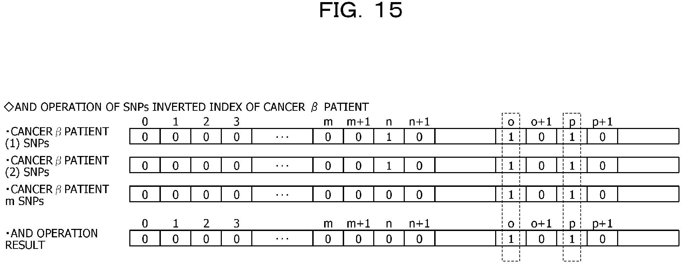

Here, the analysis of the causal relationship in each of the hospitals will be described. FIG. 14 is a diagram for explaining first causal relationship analysis at each of the hospitals according to the second embodiment, and FIG. 15 is a diagram for explaining second causal relationship analysis at each of the hospitals according to the second embodiment. Note that the analysis process to be described with reference to FIGs. 14 and 15 is executed by, for example, a generation unit 33.

Specifically, the information processing apparatus 10 of each of the hospitals obtains the personal genome of each patient and uses the method according to the first embodiment, thereby generating a bitmap-type SNPs inverted index 20 corresponding to each patient. At this time, in a case where special gene mutation is detected during gene mutation analysis of segmented genome data 13 of each personal genome, the information processing apparatus 10 stores detailed information in a dynamic dictionary. Note that codon sequence storage in an encoding part may be omitted. Then, the information processing apparatus 10 performs an AND operation (logical product) on the SNPs inverted index 20 corresponding to each patient with a disease such as cancer, thereby extracting SNPs common to individual diseases and generating an SNPs inverted index representing the causal relationship with each disease.

For example, FIG. 14 illustrates the AND operation of the SNPs inverted index 20 common to each patient diagnosed with a cancer α. Specifically, the information processing apparatus 10 performs the AND operation on the SNPs inverted index 20 of each of patients (1) to (n) with the cancer a to generate an SNPs inverted index common to the cancer α. In the example of FIG. 14, since the m-th and n-th bits are set to "1" in common for n people, the SNPs inverted index of the cancer a in which the m-th and n-th bits are set to "1" is generated.

Furthermore, the example of FIG. 15 illustrates the AND operation of the SNPs inverted index 20 common to each patient diagnosed with a cancer β. Specifically, the information processing apparatus 10 performs the AND operation on the SNPs inverted index 20 of each of patients (1) to (n) with the cancer β to generate an SNPs inverted index common to the cancer β. In the example of FIG. 15, since the o-th and p-th bits are set to "1" in common for n people, the SNPs inverted index of the cancer β in which the o-th and p-th bits are set to "1" is generated. Note that, while a comprehensive bitmap of the third base is illustrated as an example of the SNPs inverted index, the analysis may be carried out with individual bitmaps of "U", "C", "A", and "G". Furthermore, in a case where multiple adjacent SNPs affect each other, "0" clearing may be suppressed by expanding the "1" area and performing an AND operation.

Then, the information processing apparatus 10 of each of the hospitals transmits, to the integrated analysis center, the SNPs inverted index corresponding to each cancer as a causal relationship indicating the analysis result. For example, as illustrated in FIG. 13, the information processing apparatus 10 of each of the hospitals generates data having a header part, an encoding part, and a trailer part, performs Advanced Encryption Standard (AES) block encryption on each portion with multiple different passwords, and transmits it to the integrated analysis center. Note that genome ID and target cancer information are set in the header part, a codon sequence is set in the encoding part, and the SNPs inverted index representing the analyzed causal relationship, the dynamic dictionary, and the like are set in the trailer part. Furthermore, the passwords may be notified separately to the integrated analysis center, or may be determined in advance between the integrated analysis center and each of the hospitals. Note that, with regard to hashing and encryption, the adjacent prime numbers selected at the time of hashing the SNPs inverted index are stored in the header part. At that time, the header part is subject to the AES block encryption with a password different from that of the SNPs inverted index, whereby confidentiality may be further improved.

In this manner, by using the method according to the second embodiment, it becomes possible to link electronic medical records and genomes between the integrated analysis center and the hospitals to analyze the causal relationship between cancer and SNPs using the SNPs inverted index, which may be used for medical treatment such as a prophylaxis and analysis of cancer. Furthermore, SNPs of personal information included in the genome may be protected by multi-layered encryption with multiple different passwords.

### [Third Embodiment]

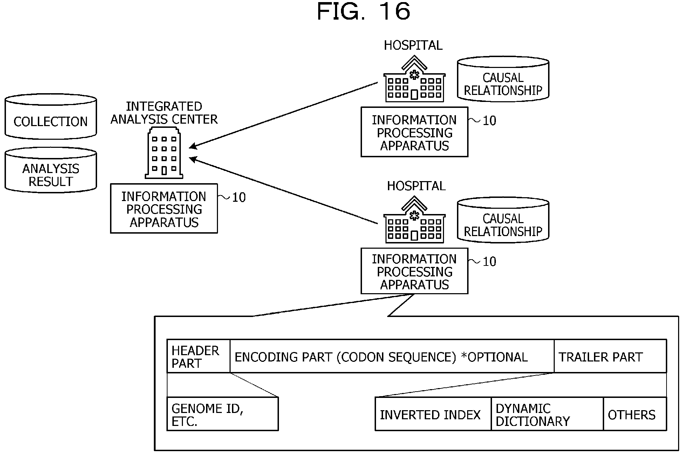

In a third embodiment, an example in which an integrated analysis center collects causal relationships of canceration from individual hospitals and comprehensively analyzes each canceration. FIG. 16 is a diagram for explaining an exemplary system configuration according to the third embodiment. In a system illustrated in FIG. 16, in a similar manner to the second embodiment, the integrated analysis center is connected to the individual hospitals in a mutually communicable manner via a network. Each of the integrated center and individual hospitals has an information processing apparatus 10 having the functions described in the first embodiment.

In such a system configuration, the information processing apparatus 10 of the integrated analysis center collects, from each of the hospitals, data associated with individual causal relationships corresponding to diseases, such as cancer, using the method described in the second embodiment, for example. Then, the information processing apparatus 10 of the integrated analysis center decodes the collected data, and analyzes integrated causal relationships common among the individual hospitals.

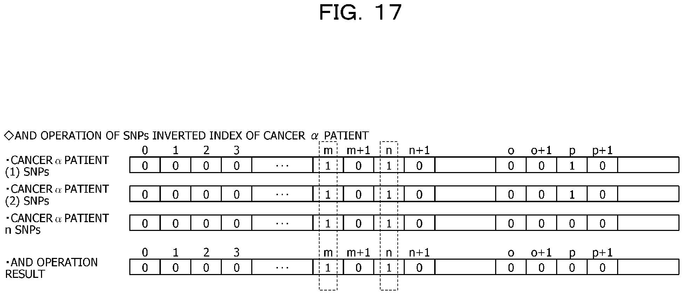

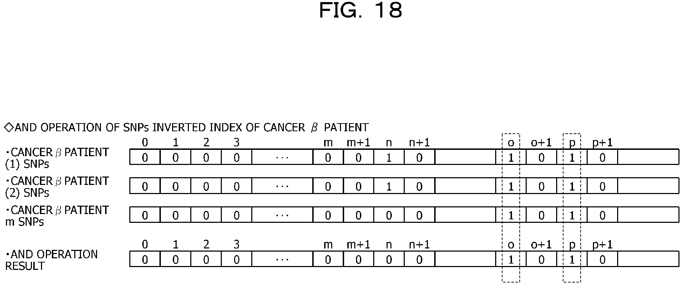

Here, the integrated analysis of the causal relationships in the integrated analysis center will be described. FIG. 17 is a diagram for explaining a first integrated analysis of causal relationships in the integrated analysis center according to the third embodiment, and FIG. 18 is a diagram for explaining a second integrated analysis of the causal relationships in the integrated analysis center according to the third embodiment. Note that the analysis process to be described with reference to FIGs. 17 and 18 is executed by, for example, a generation unit 33.

Specifically, the integrated analysis center collects causal relationship analysis results from each of the hospitals, and decodes them, thereby obtaining an SNPs inverted index corresponding to each disease, such as cancer. Then, the integrated analysis center performs, for each cancer, an AND operation (logical product) on the SNPs inverted index obtained from each of the hospitals, thereby extracting SNPs common to individual cancers and generating an inverted index for each cancer.

For example, FIG. 17 illustrates an example of performing integrated analysis of a cancer a by performing the AND operation on each SNPs inverted index 20 of the cancer α. Specifically, the information processing apparatus 10 performs the AND operation on the SNPs inverted index of the cancer a generated at each of n hospitals (hospitals x to n) to generate an SNPs inverted index common to the cancer α. In the example of FIG. 17, since the m-th and n-th bits are set to "1" in common for the n hospitals, the SNPs inverted index of the cancer a in which the m-th and n-th bits are set to "1" is generated as an integrated analysis result.

Furthermore, FIG. 18 illustrates an example of performing integrated analysis of a cancer β by performing the AND operation on each SNPs inverted index of the cancer β. Specifically, the information processing apparatus 10 performs the AND operation on the SNPs inverted index of the cancer β generated at each of the n hospitals (hospitals x to n) to generate an SNPs inverted index common to the cancer β. In the example of FIG. 18, since the o-th and p-th bits are set to "1" in common for the n hospitals, the SNPs inverted index of the cancer β in which the o-th and p-th bits are set to "1" is generated as an integrated analysis result. Note that, while a comprehensive bitmap of the third base is illustrated as an example of the SNPs inverted index, the analysis may be carried out with individual bitmaps of "U", "C", "A", and "G".

As a result, the integrated analysis center is enabled to further analyze the causal relationship between cancer and SNPs using the AND operation on the basis of data received from each of the hospitals. Furthermore, the integrated analysis center may deliver the integrated analysis result of the causal relationship between cancer and SNPs to each of the hospitals. At this time, the integrated analysis center delivers the integrated analysis result (SNPs inverted index) corresponding to each disease, such as cancer, to each of the hospitals using the transmission method described in the second embodiment.

### [Fourth Embodiment]

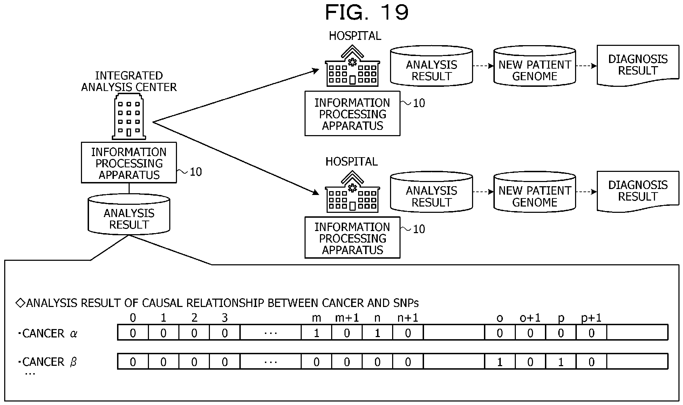

In a fourth embodiment, an example of performing canceration diagnosis at each hospital using an integrated analysis result generated in the third embodiment will be described. FIG. 19 is a diagram for explaining an exemplary system configuration according to the fourth embodiment. In a system illustrated in FIG. 19, in a similar manner to the second and third embodiments, an integrated analysis center is connected to individual hospitals in a mutually communicable manner via a network. Each of the integrated center and individual hospitals has an information processing apparatus 10 having the functions described in the first embodiment.

In such a system configuration, the integrated analysis center generates an integrated analysis result (SNPs inverted index) of causal relationships between cancer and SNPs using, for example, the method described in the third embodiment. Then, the integrated analysis center delivers the integrated analysis result to each of the hospitals using the method described in the second embodiment. Thereafter, each of the hospitals decodes the delivered integrated analysis result, and uses it to perform canceration diagnosis.

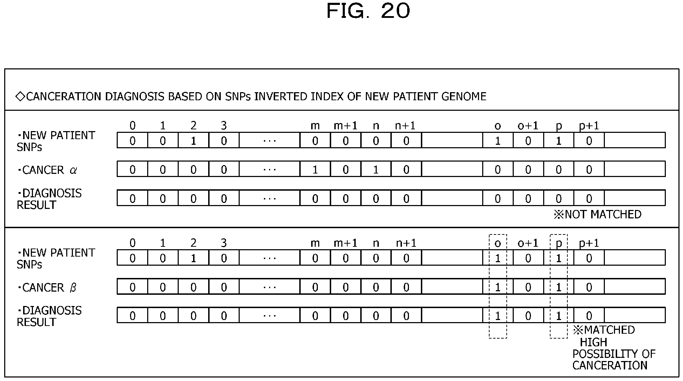

Here, the canceration diagnosis at each hospital will be described. FIG. 20 is a diagram for explaining the canceration diagnosis at each hospital using the integrated analysis result according to the fourth embodiment. The analysis process to be described with reference to FIG. 20 is executed by, for example, a generation unit 33.

As illustrated in FIG. 20, the information processing apparatus 10 of each hospital generates an SNPs inverted index 20 of a new patient using the method according to the first embodiment. Subsequently, the information processing apparatus 10 of each hospital performs an AND operation on the SNPs inverted index 20 of the new patient and the integrated analysis result (SNPs inverted index) of each cancer obtained from the integrated analysis center, thereby performing canceration diagnosis of the new patient.

In the example of FIG. 20, since all bits are "0" as a result of the AND operation of the SNPs inverted index 20 of the new patient and the SNPs inverted index of cancer a, which does not match the cancer α, the hospital diagnoses that the possibility of canceration of the cancer α is low. On the other hand, since the o-th and p-th bits are "1" as a result of the AND operation of the SNPs inverted index 20 of the new patient and the SNPs inverted index of cancer β, which matches the cancer β, the hospital diagnoses that there is a possibility of canceration of the caner β. Note that, while a comprehensive bitmap of the third base is illustrated as an example of the SNPs inverted index, the analysis may be carried out with individual bitmaps of "U", "C", "A", and "G".

In this manner, by using the method according to the fourth embodiment, it becomes possible to achieve prophylaxes and diagnoses of diseases, such as canceration, at each hospital. Furthermore, since the prophylaxes and diagnoses may be performed using the integrated SNPs inverted index using the causal relationships collected from each of the hospitals, it becomes possible to achieve resource-saving high-speed prophylaxes and diagnoses with high statistical accuracy, which may be used for early detection of cancer and the like. Note that the integrated analysis result for each cancer type generated by the integrated analysis center is an exemplary statistical inverted index.

### [Fifth Embodiment]

Although the embodiments of the present invention have been described above, the present invention may be implemented in various different modes in addition to the embodiments described above.

### [Numerical Values, etc.]

The numerical values, the number of bits, the codon codes, the number of the codon codes, the arrangement of codes, and the like used in the embodiments described above are merely examples, and may be changed in any way.

### [System]

Pieces of information including a processing procedure, a control procedure, a specific name, various types of data, and parameters described above or illustrated in the drawings may be optionally changed unless otherwise specified. Note that the codon conversion table 14 is exemplary codon conversion information, the reference codon data 17 is exemplary reference encoded data, and the SNPs inverted index 20 is exemplary gene mutation inverted index. The acquisition unit 31 is an exemplary acquisition unit, the encoding unit 32 is an exemplary generation unit that generates multiple segmented codon data, and the generation unit 33 is an exemplary generation unit that generates the gene mutation inverted index.

Furthermore, each component of each device illustrated in the drawings is functionally conceptual, and is not necessarily physically configured as illustrated in the drawings. In other words, specific forms of distribution and integration of the individual devices are not limited to those illustrated in the drawings. That is, all or a part of them may be configured by being functionally or physically distributed or integrated in optional units depending on various loads, use situations, or the like.

Moreover, all or any part of individual processing functions performed in each device may be implemented by a central processing unit (CPU) and a program analyzed and executed by the CPU, or may be implemented as hardware by wired logic.

### [Hardware]

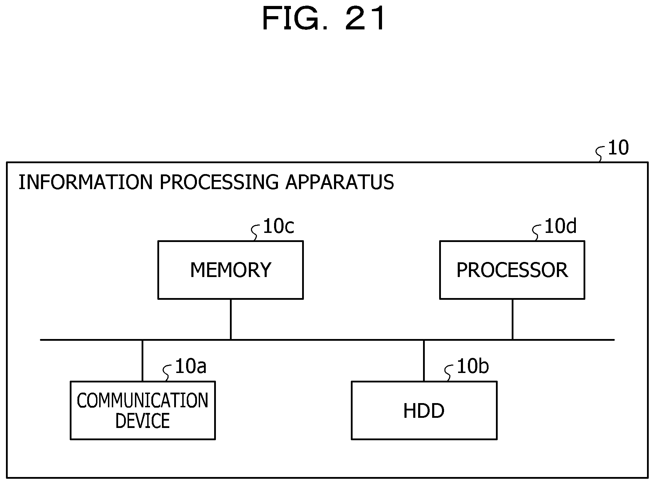

Next, an exemplary hardware configuration of the information processing apparatus 10 will be described. FIG. 21 is a diagram for explaining an exemplary hardware configuration. As illustrated in FIG. 21, the information processing apparatus 10 includes a communication device 10a, a hard disk drive (HDD) 10b, a memory 10c, and a processor 10d. Furthermore, the respective units illustrated in FIG. 21 are mutually connected by a bus or the like.

The communication device 10a is a network interface card or the like, and communicates with another server. The HDD 10b stores programs and DBs for activating the functions illustrated in FIG. 2.

The processor 10d reads a program that executes processing similar to the processing of each processing unit illustrated in FIG. 2 from the HDD 10b or the like, and loads it into the memory 10c, thereby activating a process for executing each function described with reference to FIG. 2 or the like. For example, this process executes a function similar to that of each processing unit included in the information processing apparatus 10. Specifically, the processor 10d reads a program having functions similar to those of the acquisition unit 31, the encoding unit 32, the generation unit 33, the output unit 34, and the like from the HDD 10b or the like. Then, the processor 10d executes a process for executing processing similar to that of the acquisition unit 31, the encoding unit 32, the generation unit 33, the output unit 34, or the like.

In this manner, the information processing apparatus 10 operates as an information processing apparatus that executes an information processing method by reading and executing a program. Furthermore, the information processing apparatus 10 may implement functions similar to those in the embodiments described above by reading the program described above from a recording medium with a medium reading device and executing the read program described above. Note that other programs referred to in the embodiments are not limited to being executed by the information processing apparatus 10. For example, the present invention may be similarly applied also to a case where another computer or server executes the program, or a case where such a computer and server cooperatively execute the program.

This program may be distributed via a network such as the Internet. Furthermore, this program may be recorded in a computer-readable recording medium such as a hard disk, a flexible disk (FD), a compact disc read only memory (CD-ROM), a magneto-optical disk (MO), or a digital versatile disc (DVD), and may be executed by being read from the recording medium by a computer.

### CITATION LIST

### PATENT DOCUMENT

Patent Document 1: Japanese Laid-open Patent Publication No. 2012-234558
Patent Document 2: Japanese Laid-open Patent Publication No. 2012-157283

### REFERENCE SIGNS LIST

10Information processing apparatus
11Communication unit
12Storage unit
13Segmented genome data
14Codon conversion table
15Segmented codon data
16Reference genome data
17Reference codon data
18Reference inverted index
19Partial reference codon data
20SNPs inverted index
30Control unit
31Acquisition unit
32Encoding unit
33Generation unit
34Output unit
18Reference inverted index
19Partial reference codon data
20SNPs inverted index
30Control unit
31Acquisition unit
32Encoding unit
33Generation unit
34Output unit

## Claims

1. An information processing program comprising instructions which, when executed by a computer, cause the computer to perform processing comprising:
obtaining a plurality of pieces of segmented genome data, which is genome information of a specific individual, wherein the obtaining obtains the plurality of pieces of segmented genome data, which is genome information of a cancer patient;
generating a plurality of pieces of segmented codon data obtained by encoding each of the plurality of pieces of segmented genome data in a codon unit on the basis of a codon conversion table in which a codon and a code are associated with each other, wherein the generating generates the plurality of pieces of segmented codon data that corresponds to the cancer patient;
identifying, on the basis of reference codon data obtained by encoding reference genome data to be a reference in the codon unit and each of the plurality of pieces of segmented codon data, a type and a position of an appearance of gene mutation different from the code that appears in the reference codon data among a plurality of the codes that appears in the plurality of pieces of segmented codon data, wherein the identifying identifies the type and position of the appearance of the gene mutation on the basis of the reference codon data of a healthy person and the plurality of pieces of segmented codon data that corresponds to the cancer patient;
generating a gene mutation inverted index in which the gene mutation and the type and position of the appearance of the gene mutation are associated with each other, wherein the generating generates the gene mutation inverted index that corresponds to the cancer patient using the identified type and position of the gene mutation;
calculating a logical product of each bit in which the code of the codon and the appearance position of the code of the codon are associated with each other in the gene mutation inverted index of each of a plurality of cancer patients; and
generating a statistical inverted index that represents the type and position of the gene mutation, which expresses a characteristic of the cancer patient, using a result of the logical product,
the program causing the computer to perform the process further comprising:
when the segmented genome data of a new patient to be determined is obtained and the gene mutation inverted index is generated, calculating a logical product of the gene mutation inverted index of the new patient and the statistical inverted index generated for each cancer type; and
outputting, to a person in a hospital, information indicating which cancer type the new patient corresponds to on the basis of a result of the logical product.

2. The information processing program according to claim 1, wherein the identifying identifies a position of a reference codon sequence to be searched for from a reference inverted index in which the code of the codon in the reference codon data and an appearance position of the code of the codon are associated with each other, and compares the code in the reference codon data that corresponds to the identified position with the codes in the plurality of pieces of segmented codon data that corresponds to the position to identify the type and position of the appearance of the gene mutation.

3. The information processing program according to claim 1, the program causing the computer to perform the process further comprising:
generating the gene mutation inverted index that corresponds to the segmented genome data of a patient;
generating data in which an identifier that identifies the patient, the gene mutation inverted index, and the codon conversion table are encrypted by separate encryption methods and combined; and
outputting the data.

4. An information processing method implemented by a computer, the information processing method comprising:
obtaining a plurality of pieces of segmented genome data, which is genome information of a specific individual, wherein the obtaining obtains the plurality of pieces of segmented genome data, which is genome information of a cancer patient;
generating a plurality of pieces of segmented codon data obtained by encoding each of the plurality of pieces of segmented genome data in a codon unit on the basis of a codon conversion table in which a codon and a code are associated with each other, wherein the generating generates the plurality of pieces of segmented codon data that corresponds to the cancer patient;
identifying, on the basis of reference codon data obtained by encoding reference genome data to be a reference in the codon unit and each of the plurality of pieces of segmented codon data, a type and a position of an appearance of gene mutation different from the code that appears in the reference codon data among a plurality of the codes that appears in the plurality of pieces of segmented codon data, wherein the identifying identifies the type and position of the appearance of the gene mutation on the basis of the reference codon data of a healthy person and the plurality of pieces of segmented codon data that corresponds to the cancer patient;
generating a gene mutation inverted index in which the gene mutation and the type and position of the appearance of the gene mutation are associated with each other, wherein the generating generates the gene mutation inverted index that corresponds to the cancer patient using the identified type and position of the gene mutation;
calculating a logical product of each bit in which the code of the codon and the appearance position of the code of the codon are associated with each other in the gene mutation inverted index of each of a plurality of cancer patients; and
generating a statistical inverted index that represents the type and position of the gene mutation, which expresses a characteristic of the cancer patient, using a result of the logical product,
the information processing method further comprising:
when the segmented genome data of a new patient to be determined is obtained and the gene mutation inverted index is generated, calculating a logical product of the gene mutation inverted index of the new patient and the statistical inverted index generated for each cancer type; and
outputting, to a person in a hospital, information indicating which cancer type the new patient corresponds to on the basis of a result of the logical product.

5. An information processing apparatus (10) comprising:
an acquisition unit (31) of obtaining a plurality of pieces of segmented genome data, which is genome information of a specific individual, wherein the obtaining obtains the plurality of pieces of segmented genome data, which is genome information of a cancer patient;
an encoding unit (32) of generating a plurality of pieces of segmented codon data obtained by encoding each of the plurality of pieces of segmented genome data in a codon unit on the basis of a codon conversion table in which a codon and a code are associated with each other, wherein the generating generates the plurality of pieces of segmented codon data that corresponds to the cancer patient;
a generation unit (33) of identifying, on the basis of reference codon data obtained by encoding reference genome data to be a reference in the codon unit and each of the plurality of pieces of segmented codon data, a type and a position of an appearance of gene mutation different from the code that appears in the reference codon data among a plurality of the codes that appears in the plurality of pieces of segmented codon data, wherein the identifying identifies the type and position of the appearance of the gene mutation on the basis of the reference codon data of a healthy person and the plurality of pieces of segmented codon data that corresponds to the cancer patient, generating a gene mutation inverted index in which the gene mutation and the type and position of the appearance of the gene mutation are associated with each other, wherein the generating generates the gene mutation inverted index that corresponds to the cancer patient using the identified type and position of the gene mutation, calculating a logical product of each bit in which the code of the codon and the appearance position of the code of the codon are associated with each other in the gene mutation inverted index of each of a plurality of the cancer patients, and generating a statistical inverted index that represents the type and position of the gene mutation, which expresses a characteristic of the cancer patient, using a result of the logical product, and when the segmented genome data of a new patient to be determined is obtained and the gene mutation inverted index is generated, calculating a logical product of the gene mutation inverted index of the new patient and the statistical inverted index generated for each cancer type, and
outputting, to a person in a hospital, information indicating which cancer type the new patient corresponds to on the basis of a result of the logical product.

## Patentansprüche

1. Ein Informationsverarbeitungsprogramm, das Anweisungen umfasst, die, wenn sie von einem Computer ausgeführt werden, den Computer veranlassen, eine Verarbeitung durchzuführen, die Folgendes umfasst:
Erfassen einer Vielzahl von Teilen segmentierter Genomdaten, bei denen es sich um Genominformationen eines bestimmten Individuums handelt, wobei das Erfassen die Vielzahl von Teilen segmentierter Genomdaten erfasst, bei denen es sich um Genominformationen eines Krebspatienten handelt;
Erzeugen einer Vielzahl von Teilen segmentierter Codondaten, erhalten durch Kodieren jedes der Vielzahl von Teilen segmentierter Genomdaten in einer Codon-Einheit auf der Grundlage einer Codon-Zuordnungstabelle, in der ein Codon und ein Code einander zugeordnet sind, wobei das Erzeugen die Vielzahl von Teilen segmentierter Codondaten erzeugt, die dem Krebspatienten entspricht;
Identifizieren, auf der Grundlage von Referenz-Codondaten, die erhalten werden durch Kodieren von Referenz-Genomdaten, die als Referenz dienen, in der Codon-Einheit, und jedes der Vielzahl von Teilen segmentierter Codondaten, eines Typs und einer Position eines Auftretens einer Genmutation, das sich von dem Code unterscheidet, der in den Referenz-Codondaten erscheint, unter einer Vielzahl der Codes, die in der Vielzahl von Teilen segmentierter Codondaten erscheinen, wobei das Identifizieren den Typ und die Position des Auftretens der Genmutation auf der Grundlage der Referenz-Codondaten einer gesunden Person und der Vielzahl von Teilen segmentierter Codondaten, die dem Krebspatienten entspricht, identifiziert;
Erzeugen eines invertierten Index für Genmutationen, in dem die Genmutation sowie der Typ und die Position des Auftretens der Genmutation einander zugeordnet sind, wobei das Erzeugen den invertierten Index für Genmutationen erzeugt, der dem Krebspatienten entspricht, unter Verwendung des identifizierten Typs und der identifizierten Position der Genmutation;
Berechnung eines logischen Produkts jedes Bits, in dem der Codoncode und die Auftretensposition des Codoncodes im invertierten Index für Genmutationen jedes der Vielzahl von Krebspatienten einander zugeordnet sind; und
Erzeugen eines statistischen invertierten Index, der den Typ und die Position der Genmutation darstellt, die eine Eigenschaft des Krebspatienten ausdrücken, unter Verwendung eines Ergebnisses des logischen Produkts,
wobei das Programm den Computer veranlasst, den Prozess auszuführen, der weiter Folgendes umfasst:
wenn die zu bestimmenden segmentierten Genomdaten eines neuen Patienten erfasst werden und der invertierte Index für Genmutationen erzeugt wird, Berechnen eines logischen Produkts des invertierten Index für Genmutationen des neuen Patienten und des statistischen invertierten Index, der für jeden Krebstyp erzeugt wurde; und
Ausgeben von Informationen an eine Person in einem Krankenhaus, die angeben, welchem Krebstyp der neue Patient entspricht, auf der Grundlage eines Ergebnisses des logischen Produkts.

2. Informationsverarbeitungsprogramm, nach Anspruch 1, wobei das Identifizieren eine Position einer zu suchenden Referenz-Codonsequenz aus einem Referenz-invertierten Index identifiziert, in dem der Codoncode in den Referenz-Codondaten und eine Auftretensposition des Codoncodes einander zugeordnet sind, nd den Code in den Referenz-Codondaten, der der identifizierten Position entspricht, mit den Codes in der Vielzahl von Teilen segmentierter Codondaten vergleicht, die der Position entsprechen, um den Typ und die Position des Auftretens der Genmutation zu identifizieren.

3. Informationsverarbeitungsprogramm, nach Anspruch 1, wobei das Programm den Computer veranlasst, den Prozess auszuführen, der weiter Folgendes umfasst:
Erzeugen des invertierten Index für Genmutationen, der den segmentierten Genomdaten eines Patienten entspricht;
Erzeugen von Daten, in denen ein Identifikator, der den Patienten identifiziert, der invertierte Index für Genmutationen und die Codon-Zuordnungstabelle durch separate Verschlüsselungsverfahren verschlüsselt und kombiniert werden; und
Ausgeben der Daten.

4. Computerimplementiertes Informationsverarbeitungsverfahren, wobei das Informationsverarbeitungsverfahren Folgendes umfasst:
Erfassen einer Vielzahl von Teilen segmentierter Genomdaten, bei denen es sich um Genominformationen eines bestimmten Individuums handelt, wobei das Erfassen die Vielzahl von Teilen segmentierter Genomdaten erfasst, bei denen es sich um Genominformationen eines Krebspatienten handelt;
Erzeugen einer Vielzahl von Teilen segmentierter Codondaten, erhalten durch Kodieren jedes der Vielzahl von Teilen segmentierter Genomdaten in einer Codon-Einheit auf der Grundlage einer Codon-Zuordnungstabelle, in der ein Codon und ein Code einander zugeordnet sind, wobei das Erzeugen die Vielzahl von Teilen segmentierter Codondaten erzeugt, die dem Krebspatienten entspricht;
Identifizieren, auf der Grundlage von Referenz-Codondaten, die erhalten werden durch Kodieren von Referenz-Genomdaten, die als Referenz dienen, in der Codon-Einheit, und jedes der Vielzahl von Teilen segmentierter Codondaten, eines Typs und einer Position eines Auftretens einer Genmutation, das sich von dem Code unterscheidet, der in den Referenz-Codondaten erscheint, unter einer Vielzahl der Codes, die in der Vielzahl von Teilen segmentierter Codondaten erscheinen, wobei das Identifizieren den Typ und die Position des Auftretens der Genmutation auf der Grundlage der Referenz-Codondaten einer gesunden Person und der Vielzahl von Teilen segmentierter Codondaten, die dem Krebspatienten entspricht, identifiziert;
Erzeugen eines invertierten Index für Genmutationen, in dem die Genmutation sowie der Typ und die Position des Auftretens der Genmutation einander zugeordnet sind, wobei das Erzeugen den invertierten Index für Genmutationen erzeugt, der dem Krebspatienten entspricht, unter Verwendung des identifizierten Typs und der identifizierten Position der Genmutation;
Berechnung eines logischen Produkts jedes Bits, in dem der Codoncode und die Auftretensposition des Codoncodes im invertierten Index für Genmutationen jedes der Vielzahl von Krebspatienten einander zugeordnet sind; und
Erzeugen eines statistischen invertierten Index, der den Typ und die Position der Genmutation darstellt, die eine Eigenschaft des Krebspatienten ausdrücken, unter Verwendung eines Ergebnisses des logischen Produkts,
wobei das Informationsverarbeitungsverfahren weiter Folgendes umfasst:
wenn die zu bestimmenden segmentierten Genomdaten eines neuen Patienten erfasst werden und der invertierte Index für Genmutationen erzeugt wird, Berechnen eines logischen Produkts des invertierten Index für Genmutationen des neuen Patienten und des statistischen invertierten Index, der für jeden Krebstyp erzeugt wurde; und
Ausgeben von Informationen an eine Person in einem Krankenhaus, die angeben, welchem Krebstyp der neue Patient entspricht, auf der Grundlage eines Ergebnisses des logischen Produkts.

5. Informationsverarbeitungseinrichtung (10) umfassend:
eine Erfassungseinheit (31) zum Erfassen einer Vielzahl von Teilen segmentierter Genomdaten, bei denen es sich um Genominformationen eines bestimmten Individuums handelt, wobei das Erfassen die Vielzahl von Teilen segmentierter Genomdaten erfasst, bei denen es sich um Genominformationen eines Krebspatienten handelt;
eine Kodierungseinheit (32) zum Erzeugen einer Vielzahl von Teilen segmentierter Codondaten, erhalten durch Kodieren jedes der Vielzahl von Teilen segmentierter Genomdaten in einer Codon-Einheit auf der Grundlage einer Codon-Zuordnungstabelle, in der ein Codon und ein Code einander zugeordnet sind, wobei das Erzeugen die Vielzahl von Teilen segmentierter Codondaten erzeugt, die dem Krebspatienten entspricht;
eine Erzeugungseinheit (33) zum Identifizieren, auf der Grundlage von Referenz-Codondaten, die erhalten werden durch Kodieren von Referenz-Genomdaten, die als Referenz dienen, in der Codon-Einheit, und jedes der Vielzahl von Teilen segmentierter Codondaten, eines Typs und einer Position eines Auftretens einer Genmutation, das sich von dem Code unterscheidet, der in den Referenz-Codondaten erscheint, unter einer Vielzahl der Codes, die in der Vielzahl von Teilen segmentierter Codondaten erscheinen, wobei das Identifizieren den Typ und die Position des Auftretens der Genmutation auf der Grundlage der Referenz-Codondaten einer gesunden Person und der Vielzahl von Teilen segmentierter Codondaten, die dem Krebspatienten entspricht, identifiziert, Erzeugen eines invertierten Index für Genmutationen, in dem die Genmutation sowie der Typ und die Position des Auftretens der Genmutation einander zugeordnet sind,
wobei das Erzeugen den invertierten Index für Genmutationen erzeugt, der dem Krebspatienten entspricht, unter Verwendung des identifizierten Typs und der identifizierten Position der Genmutation, Berechnen eines logischen Produkts für jedes Bit, in dem der Codoncode und die Auftretensposition des Codoncodes im invertierten Index für Genmutationen jedes der Vielzahl von Krebspatienten einander zugeordnet sind, und Erzeugen eines statistischen invertierten Index, der den Typ und die Position der Genmutation darstellt, die eine Eigenschaft des Krebspatienten ausdrücken, unter Verwendung eines Ergebnisses des logischen Produkts, und wenn die zu bestimmenden segmentierten Genomdaten eines neuen Patienten erfasst werden und der invertierte Index für Genmutationen erzeugt wird, Berechnen eines logischen Produkts des invertierten Index für Genmutationen des neuen Patienten und des statistischen invertierten Index, der für jeden Krebstyp erzeugt wurde, und
Ausgeben von Informationen an eine Person in einem Krankenhaus, die angeben, welchem Krebstyp der neue Patient entspricht, auf der Grundlage eines Ergebnisses des logischen Produkts.

## Revendications

1. Programme de traitement d'informations comprenant des instructions qui, lorsqu'elles sont exécutées par un ordinateur, amènent l'ordinateur à réaliser un traitement comprenant :
l'obtention d'une pluralité d'éléments de données génomiques segmentées, qui sont des informations génomiques d'un individu spécifique, dans lequel l'obtention permet d'obtenir la pluralité d'éléments de données génomiques segmentées, qui sont des informations génomiques d'un patient atteint d'un cancer ;
la génération d'une pluralité d'éléments de données de codon segmentées obtenues par codage de chacun de la pluralité d'éléments de données génomiques segmentées dans une unité de codon sur la base d'une table de conversion de codon dans laquelle un codon et un code sont associés entre eux, dans lequel la génération permet de générer la pluralité d'éléments de données de codon segmentées qui correspondent au patient atteint d'un cancer ;
l'identification, sur la base de données de codon de référence obtenues par codage de données génomiques de référence servant de référence dans l'unité de codon et de chacun de la pluralité d'éléments de données de codon segmentées, d'un type et d'une position d'une apparition d'une mutation génétique différente du code qui apparaît dans les données de codon de référence parmi une pluralité des codes qui apparaissent dans la pluralité d'éléments de données de codon segmentées, dans lequel l'identification permet d'identifier le type et la position de l'apparition de la mutation génétique sur la base des données de codon de référence d'une personne en bonne santé et de la pluralité d'éléments de données de codon segmentées qui correspondent au patient atteint d'un cancer ;
la génération d'un index inversé de mutation génétique dans lequel la mutation génétique et le type et la position de l'apparition de la mutation génétique sont associés entre eux, dans lequel la génération permet de générer l'index inversé de mutation génétique qui correspond au patient atteint d'un cancer en utilisant le type et la position identifiés de la mutation génétique ;
le calcul d'un produit logique de chaque bit dans lequel le code du codon et la position d'apparition du code du codon sont associés entre eux dans l'index inversé de mutation génétique de chacun d'une pluralité de patients atteints d'un cancer ; et
la génération d'un index inversé statistique qui représente le type et la position de la mutation génétique, qui exprime une caractéristique du patient atteint d'un cancer, en utilisant un résultat du produit logique,
le programme amenant l'ordinateur à réaliser le processus comprenant en outre :
lorsque les données génomiques segmentées d'un nouveau patient à déterminer sont obtenues et que l'index inversé de mutation génétique est généré, le calcul d'un produit logique de l'index inversé de mutation génétique du nouveau patient et de l'index inversé statistique généré pour chaque type de cancer ; et
la délivrance en sortie, à une personne dans un hôpital, d'informations indiquant à quel type de cancer correspond le nouveau patient sur la base d'un résultat du produit logique.

2. Programme de traitement d'informations selon la revendication 1, dans lequel l'identification permet d'identifier une position d'une séquence de codon de référence à rechercher à partir d'un index inversé de référence dans lequel le code du codon dans les données de codon de référence et une position d'apparition du code du codon sont associés entre eux, et de comparer le code dans les données de codon de référence qui correspondent à la position identifiée aux codes dans la pluralité d'éléments de données de codon segmentées qui correspondent à la position pour identifier le type et la position de l'apparition de la mutation génétique.

3. Programme de traitement d'informations selon la revendication 1, le programme amenant l'ordinateur à réaliser le processus comprenant en outre :
la génération de l'index inversé de mutation génétique qui correspond aux données génomiques segmentées d'un patient ;
la génération de données dans lesquelles un identifiant qui identifie le patient, l'index inversé de mutation génétique et la table de conversion de codon sont chiffrés par des méthodes de chiffrement distinctes et sont combinés ; et
la délivrance en sortie des données.

4. Procédé de traitement d'informations mis en œuvre par un ordinateur, le procédé de traitement d'informations comprenant :
l'obtention d'une pluralité d'éléments de données génomiques segmentées, qui sont des informations génomiques d'un individu spécifique, dans lequel l'obtention permet d'obtenir la pluralité d'éléments de données génomiques segmentées, qui sont des informations génomiques d'un patient atteint d'un cancer ;
la génération d'une pluralité d'éléments de données de codon segmentées obtenues par codage de chacun de la pluralité d'éléments de données génomiques segmentées dans une unité de codon sur la base d'une table de conversion de codon dans laquelle un codon et un code sont associés entre eux, dans lequel la génération permet de générer la pluralité d'éléments de données de codon segmentées qui correspondent au patient atteint d'un cancer ;
l'identification, sur la base de données de codon de référence obtenues par codage de données génomiques de référence servant de référence dans l'unité de codon et de chacun de la pluralité d'éléments de données de codon segmentées, d'un type et d'une position d'une apparition d'une mutation génétique différente du code qui apparaît dans les données de codon de référence parmi une pluralité des codes qui apparaissent dans la pluralité d'éléments de données de codon segmentées, dans lequel l'identification permet d'identifier le type et la position de l'apparition de la mutation génétique sur la base des données de codon de référence d'une personne en bonne santé et de la pluralité d'éléments de données de codon segmentées qui correspondent au patient atteint d'un cancer ;
la génération d'un index inversé de mutation génétique dans lequel la mutation génétique et le type et la position de l'apparition de la mutation génétique sont associés entre eux, dans lequel la génération permet de générer l'index inversé de mutation génétique qui correspond au patient atteint d'un cancer en utilisant le type et la position identifiés de la mutation génétique ;
le calcul d'un produit logique de chaque bit dans lequel le code du codon et la position d'apparition du code du codon sont associés entre eux dans l'index inversé de mutation génétique de chacun d'une pluralité de patients atteints d'un cancer ; et
la génération d'un index inversé statistique qui représente le type et la position de la mutation génétique, qui exprime une caractéristique du patient atteint d'un cancer, en utilisant un résultat du produit logique,
le procédé de traitement d'informations comprenant en outre :
lorsque les données génomiques segmentées d'un nouveau patient à déterminer sont obtenues et que l'index inversé de mutation génétique est généré, le calcul d'un produit logique de l'index inversé de mutation génétique du nouveau patient et de l'index inversé statistique généré pour chaque type de cancer ; et
la délivrance en sortie, à une personne dans un hôpital, d'informations indiquant à quel type de cancer correspond le nouveau patient sur la base d'un résultat du produit logique.

5. Appareil (10) de traitement d'informations comprenant :
une unité d'acquisition (31) destinée à obtenir une pluralité d'éléments de données génomiques segmentées, qui sont des informations génomiques d'un individu spécifique, dans lequel l'obtention permet d'obtenir la pluralité d'éléments de données génomiques segmentées, qui sont des informations génomiques d'un patient atteint d'un cancer ;
une unité de codage (32) destinée à générer une pluralité d'éléments de données de codon segmentées obtenues par codage de chacun de la pluralité d'éléments de données génomiques segmentées dans une unité de codon sur la base d'une table de conversion de codon dans laquelle un codon et un code sont associés entre eux, dans lequel la génération permet de générer la pluralité d'éléments de données de codon segmentées qui correspondent au patient atteint d'un cancer ;
une unité de génération (33) destinée à identifier, sur la base de données de codon de référence obtenues par codage de données génomiques de référence servant de référence dans l'unité de codon et chacun de la pluralité d'éléments de données de codon segmentées, un type et une position d'une apparition d'une mutation génétique différente du code qui apparaît dans les données de codon de référence parmi une pluralité des codes qui apparaissent dans la pluralité d'éléments de données de codon segmentées, dans lequel l'identification permet d'identifier le type et la position de l'apparition de la mutation génétique sur la base des données de codon de référence d'une personne en bonne santé et de la pluralité d'éléments de données de codon segmentées qui correspondent au patient atteint d'un cancer, la génération d'un index inversé de mutation génétique dans lequel la mutation génétique et le type et la position de l'apparition de la mutation génétique sont associés entre eux, dans lequel la génération permet de générer l'index inversé de mutation génétique qui correspond au patient atteint d'un cancer en utilisant le type et la position identifiés de la mutation génétique, le calcul d'un produit logique de chaque bit dans lequel le code du codon et la position d'apparition du code du codon sont associés entre eux dans l'index inversé de mutation génétique de chacun d'une pluralité de patients atteints d'un cancer, et la génération d'un index inversé statistique qui représente le type et la position de la mutation génétique, qui exprime une caractéristique du patient atteint d'un cancer, en utilisant un résultat du produit logique, et lorsque les données génomiques segmentées d'un nouveau patient à déterminer sont obtenues et que l'index inversé de mutation génétique est généré, le calcul d'un produit logique de l'index inversé de mutation génétique du nouveau patient et de l'index inversé statistique généré pour chaque type de cancer, et
la délivrance en sortie, à une personne dans un hôpital, d'informations indiquant à quel type de cancer correspond le nouveau patient sur la base d'un résultat du produit logique.
